Europäisches Patentamt

European Patent Office

Office européen des brevets

(19)

(11) Numéro de publication: **0 566 685 B1**

(12)

# FASCICULE DE BREVET EUROPEEN

(45) Date de publication du fascicule du brevet: **13.09.95**

(51) Int. Cl.⁶: **C12Q 1/68**, //C07H21/04, C12N15/13

(21) Numéro de dépôt: **92904718.1**

(22) Date de dépôt: **09.01.92**

(86) Numéro de dépôt internationale : **PCT/FR92/00014**

(87) Numéro de publication internationale : **WO 92/12260 (23.07.92 92/19)**

(54) **PROCEDE DE DESCRIPTION DES REPERTOIRES D'ANTICORPS (Ab) ET DES RECEPTEURS DES CELLULES T (TcR) DU SYSTEME IMMUNITAIRE D'UN INDIVIDU.**

(30) Priorité: **09.01.91 FR 9100189**

(43) Date de publication de la demande: **27.10.93 Bulletin 93/43**

(45) Mention de la délivrance du brevet: **13.09.95 Bulletin 95/37**

(84) Etats contractants désignés: **BE CH DE ES FR GB IT LI NL SE**

(56) Documents cités: **WO-A-90/04648**

**ANALYTICAL BIOCHEMISTRY, vol. 162, 1987; pp. 156-159&NUM;**

**NATURE, vol. 341, 07 septembre 1989; D.M. ASARNOW et al., pp. 60-62&NUM;**

**CHEMICAL ABSTRACTS, vol. 111, 1989, Columbus, OH (US); M.E. ROTH et al., p. 176, no. 147822p&NUM;**

**NATURE, vol. 339, 29 juin 1989, Y. TAKAGAKI et al., pp. 712-714&NUM;**

(73) Titulaire: **INSTITUT NATIONAL DE LA SANTE ET DE LA RECHERCHE MEDICALE (INSERM) (E.P.S.T.)**
**101, rue de Tolbiac**
**F-75013 Paris (FR)**

Titulaire: **INSTITUT PASTEUR**
**28, rue du Docteur Roux**
**F-75724 Paris Cédex 15 (FR)**

(72) Inventeur: **KOURILSKY, Philippe**
**26, rue de Montpensier**
**F-75001 Paris (FR)**
Inventeur: **PANNETIER, Christophe**
**12, boulevard Vincent-Auriol**
**F-75013 Paris (FR)**
Inventeur: **COCHET, Madeleine**
**15, avenue Gabriel-Péri**
**F-92260 Fontenay-aux-Roses (FR)**

Rank Xerox (UK) Business Services
(3.10/3.09/3.3.3)

(74) Mandataire: **Warcoin, Jacques et al**
**Cabinet Régimbeau,**
**26, avenue Kléber**
**F-75116 Paris (FR)**

## Description

La présente invention concerne un procédé permettant la description du répertoire immunitaire d'un individu et, ainsi, la mise en évidence et/ou le suivi de certains états pathologiques.

Une caractéristique essentielle du système immunitaire est la capacité de reconnaître spécifiquement un grand nombre d'antigènes. Chez les vertébrés, les lymphocytes T et B assurent principalement cette fonction de reconnaissance, au moyen d'au moins trois complexes moléculaires transmembranaires ; l'immunoglobuline pour les cellules B, et les deux récepteurs T pour les lymphocytes T : le récepteur $\alpha\beta$ et le récepteur $\gamma\delta$ qui définissent ainsi deux sous-populations parmi l'ensemble des lymphocytes T (Wilson et al., 1988 ; Raulet, 1989).

A la formidable variété des antigènes à reconnaître, correspond une très grande diversité potentielle de ces trois types de récepteurs. En effet, ces trois complexes moléculaires, de structure voisine, sont composés de deux (pour chacun des récepteurs T) ou quatre (pour les immunoglobines) chaînes peptidiques dont les domaines NH2-terminaux sont très variables. C'est l'existence d'une interaction forte entre un déterminant antigénique donné et le site constitué par ces domaines variables, qui constitue la traduction à l'échelle moléculaire du phénomène de reconnaissance. C'est ainsi que les informations contenues dans le génome d'une souris lui permettent de produire potentiellement au moins $10^{11}$ immunoglobulines de région variable différente, $10^{15}$ récepteurs $T\alpha\beta$ différents, et $10^{18}$ récepteurs $T\gamma\delta$ différents.

Ainsi émerge la notion de répertoire : l'ensemble des régions variables des immunoglobulines présentes à un instant donné dans un organisme constitue le répertoire actuel des immunoglobulines. De même, l'ensemble des régions variables des récepteurs $T\gamma\delta$ pouvant être éventuellement produites par un génome de souris constitue le répertoire potentiel des lymphocytes $\gamma\delta$.

Le système immunitaire contient donc, en fait, trois répertoires complexes, du fait que le répertoire des récepteurs T doit être subdivisé en répertoire des récepteurs $T\alpha\beta$ et des récepteurs $T\gamma\delta$.

Les mécanismes qui permettent de produire l'immense diversité des récepteurs des lymphocytes T et B sont maintenant bien connus (Tonegawa, 1983). La région variable d'une immunoglobuline ou d'un récepteur T est formée par les domaines NH2-terminaux de deux chaînes peptidiques. Les gènes codant pour ces deux protéines sont le résultat de réarrangements somatiques qui juxtaposent un segment V, un ou deux segments D selon les chaînes, et un segment J. Le nombre de segments V, D et J disponibles dans les différents loci fournit une première source de diversité dite diversité combinatoire (cf tableau 1). De plus, l'imprécision des jonctions entre deux de ces segments (jonctions V-D, D-D, D-J ou V-J) introduit une deuxième source de diversité dite diversité jonctionnelle, puisque d'une part, chacune des deux extrémités juxtaposées peut se trouver amputée de quelques bases, et que d'autre part, peuvent être insérés des nucléotides au site de la jonction. Enfin, dans le cas des gènes codant pour les immunoglobulines, des mutations somatiques peuvent se produire dans le deuxième exon du gène réarrangé, ce qui constitue une troisième source de diversité.

Selon le type de récepteur $\alpha\beta$ , $\gamma\delta$ ou immunoglobuline, chacune de ces trois sources de diversité est une composante plus ou moins importante de la diversité totale. Ainsi, alors que le nombre de segments V, D et J disponibles est le plus faible dans le cas du récepteur $\gamma\delta$, l'étendue du répertoire de ce récepteur reste potentiellement très grande. Ceci provient essentiellement du fait que le gène codant la chaine $\delta$ peut comprendre zéro, un ou deux éléments D, qui peuvent de plus être lus dans les trois phases de lecture, alors que les chaînes $V_H$ et $\beta$ comptent un et un seul élément D. En fait, la diversité du répertoire des récepteurs $\gamma\delta$ peut être décrite de la façon suivante : il semble qu'un faible nombre de segments V et J soit disponible, mais le nombre de segments V de la chaîne $\delta$ est encore mal connu et il existe une très grande diversité jonctionnelle potentielle qui se traduit par une variation importante de la longueur du deuxième exon du gène réarrangé, surtout dans le cas du gène codant pour la chaîne $\delta$ (Raulet, 1989).

La fonction des lymphocytes $T\alpha\beta$ est relativement bien connue : ils interviennent dans la cytolyse par les cellules tueuses, dans les réactions qui règlent la synthèse des anticorps et dans des phénomènes inflammatoires. La fonction des lymphocytes $T\gamma\delta$ est encore mal comprise : on admet en général qu'outre leur rôle probable dans l'ontogénie du système immunitaire, les cellules $T\gamma\delta$ participent à la surveillance immunitaire. Quant aux diverses fonctions des anticorps, elles sont relativement bien connues et ne seront pas rappelées ici.

Nul n'est aujourd'hui capable de décrire l'ensemble des anticorps et des récepteurs T (i.e. les répertoires des Ab et des TcR) exprimés à un moment donné dans un individu. Il s'agit d'une tâche formidable puisque chacun des répertoires comporte vraisemblablement des millions de molécules différentes. On ne dispose encore que d'un faible nombre de réactifs capables de reconnaître spécifiquement tel ou tel élément de tel répertoire. On peut certes opérer plus finement en déterminant la séquence d'un certain nombre de gènes exprimés. Toutefois, des considérations pratiques font qu'il n'est guère concevable d'analyser, en routine, plus d'une dizaine ou, peut-être, d'une centaine de gènes, et l'opération est coûteuse et très longue. En bref, on ne décrit, à l'heure actuelle, les répertoires d'anticorps et de récepteurs T qu'au moyen d'un petit nombre de paramètres. On ne dispose donc pas de méthodes qui permettent d'analyser rapidement et efficacement des situations physiologiques et pathologiques liées à l'état de ces répertoires. Par exemple, il est clair que ces répertoires varient au cours d'une immunisation volontaire (vaccin), ou au cours de l'infection par des microorganismes pathogènes, ou encore dans l'évolution de pathologies auto-immunes. Dans ce dernier cas, il y a de nombreuses raisons de penser que la prédisposition à ces maladies est le reflet d'une certaine composition des répertoires. Il est donc très probable qu'une bonne méthode d'analyse des répertoires aurait des retombées médicales, et pourrait fonder des techniques d'analyse médicale et de diagnostic.

Une méthode maintenant très utilisée dans le but d'étudier la diversité et la distribution des trois répertoires immunoglobulines, récepteurs $\alpha\beta$ et $\gamma\delta$, est celle de l'amplification par PCR (Polymerase Chain Reaction). Cette technique consiste à amplifier l'ADN génomique ou l'ADN complémentaire avec une série de couples d'amorces spécifiques (V,C) ou (V,J) et le cas échéant, de cioner puis de séquencer les produits d'amplification obtenus (Takagaki et al., 1989) ; Asarnow et al., 1989). Cette méthode puissante n'est pas exempte d'artefacts. Sans même parler des problèmes liés à la quantification, il semble par exemple hasardeux de déduire de deux amplifications réalisées avec deux couples d'amorces différents, (V1, C) et (V2, C) par exemple, un usage préférentiel d'un segment par rapport à l'autre dans la population étudiée (Rajasekar et al., 1990). Dans l'état actuel de la technique, il est difficile de déterminer par cette méthode le taux d'usage des différents segments V. D'autre part, le clonage des produits d'amplification en vue de déterminer leur séquence peut également générer des artefacts. Par exemple, une certaine proportion de ces produits sont en fait des hétéroduplexes (si la population amplifiée est hétérogène) dont on ne peut prédire comment il seront "réparés" après transformation de la bactérie (Abastado et al., 1984, 1987).

Un perfectionnement récent est l'amplification suivant la technique de la PCR ancrée, qui consiste à réaliser l'amplification d'une population hétérogène d'ADN complémentaires à l'aide d'un couple unique d'amorces, l'une s'hybridant dans la région constante C, l'autre avec une séquence identique ajoutée en 3' de tous les brins d'ADN complémentaires. On peut donc espérer que le rendement de l'amplification ne dépende pas du segment Y utilisé dans le réarrangement.

Récemment, une méthode assez sensible permettant d'évaluer le taux d'usage des différents segments V dans une population de transcrits hétérogènes a été développée (Okada et Weissman, 1989 ; Singer et al., 1990). Cette méthode précise et reproductible a l'avantage d'être simple conceptuellement donc d'introduire peu de biais. Cependant, elle ne permet ni de définir simultanément l'usage V et l'usage J, ni de déterminer si le transcrit révélé est en phase.

Parallèlement à l'amplification à partir de populations polyclonales, une autre démarche largement utilisée a consisté à construire des banques d'hybridomes ou de lignées T clonales, puis de caractériser au niveau moléculaire les anticorps ou les récepteurs T exprimés. Cette méthode est évidemment difficile à mettre en oeuvre à grande échelle. Elle introduit des biais difficiles à évaluer, lors des étapes de fusion ou de clonage, mais est la seule technique actuelle permettant de déterminer simultanément la séquence des deux chaînes d'une récepteur T ou de déterminer un répertoire d'une spécificité connue.

Il faut noter que les méthodes citées plus haut caractérisent toutes le répertoire à partir de l'ARN messager et non pas de la protéine, de sorte que tout contrôle post-transcriptionnel est méconnu. L'utilisation d'anticorps monoclonaux et de la cytofluorometrie en flux permet d'analyser le répertoire au niveau du récepteur même et donne simultanément de multiples renseignements sur le phénotype des cellules étudiées. Cependant, cette méthode est assez peu sensible, et surtout, ne permet pas d'étudier dans le détail le répertoire puisqu'il est impossible, faute de réactifs, d'accéder à la diversité jonctionnelle du répertoire. Elle nécessite de plus de disposer d'une batterie importante d'anticorps monoclonaux bien caractérisés.

La présente invention a plus particulièrement pour objet un procédé permettant la description du répertoire immunitaire d'un individu, qui puisse être facilement automatisé, prenne en compte un très grand nombre de paramètres et soit dépourvu des biais des procédés décrits précédemment.

Plus précisément, la présente invention concerne un procédé de description des répertoires d'anticorps (Ab) et de récepteurs des cellules T (TcR) du système immunitaire d'un individu, caractérisé en ce que :

- à partir d'un prélèvement biologique, on effectue, le cas échéant, la transcription réverse des ARNm qu'il contient, on effectue ensuite, sur le produit de transcription ou directement sur l'ADN extrait de l'échantillon, des amplifications séparées par une méthode de type PCR pour chaque couple d'amorce V,C, V correspondant à un segment variable du répertoire en cause et C s'hybridant au segment constant du répertoire étudié,

- sur chacun de ces produits d'amplification on effectue, pour chaque segment J du répertoire, une étape d'élongation utilisant comme amorce un oligonucléotide marqué spécifique de ce segment J et le produit d'amplification comme matrice,
- pour chaque produit d'élongation correspondant à un triplet (V,C)J ainsi obtenu on fait apparaître la taille des différents produits d'élongation, au nucléotide près, par les procédés généralement utilisés pour la détermination des séquences d'ADN,
- la description des répertoires correspond pour chaque élément du répertoire, à un triplet (V,C)J et à la taille de l'élément.

Une alternative consiste à extraire l'ADN de l'échantillon biologique et à pratiquer l'amplification sur l'ADN dénaturé, en utilisant le fait que les réarrangements productifs rapprochent le segment variable de la région constante, de sorte que seuls les segments variables utilisés servent efficacement dans l'amplification.

Le procédé selon l'invention peut être utilisé aussi bien pour décrire l'un des trois répertoires ou bien les trois ou seulement deux d'entre eux. Dans ce qui suit, on décrira particulièrement le répertoire $T\gamma\delta$ mais la même méthodologie est utilisable pour le répertoire $T\alpha\beta$ et les immunoglobulines.

Par "description du répertoire" on entend désigner aussi bien un état sous forme de tableau, bi ou tridimensionnel, qu'une représentation graphique et/ou des reproductions de gel par exemple.

En effet, l'un des intérêts de la présente invention est de pouvoir comparer ces répertoires, en particulier à des états de répertoire type pouvant être liés à des physiologies ou des pathologies déterminées ou bien de suivre l'évolution de ces répertoires dans l'apparition et/ou l'évolution de certaines maladies telles que certains cancers, maladies autoimmunes ou SIDA. Par exemple, au cours de la réaction immunitaire qui survient dans au moins quelques types de cancers, la détection rapide des lymphocytes infiltrant les tumeurs (TIL), l'observation de leur évolution, ainsi que du reste du répertoire au cours d'un traitement par immunothérapie pourrait se révéler une approche intéressante. On peut envisager d'appliquer la puissance de la méthode à l'étude de l'évolution du répertoire au cours du développement d'une maladie autoimmune. On sait en effet que dans nombre de maladies autoimmunes, les lymphocytes T tiennent une importance de premier plan. Si l'on devait, à la suite d'études réalisées dans les quelques modèles animaux disponibles, observer des corrélations strictes entre certaines de ces pathologies et un état ou une suite d'états du répertoire mesurés selon la méthode présentée, il serait alors parfaitement envisageable de donner une valeur prédictive à cette méthode d'analyse, et par conséquent une valeur de diagnostic plus précoce que les tests actuellement disponibles. Ce procédé peut donc devenir un outil d'investigation et d'analyse puissant pour l'étude et la surveillance du système immuntaire.

Les méthodologies mises en oeuvre dans ce procédé sont connues dans leurs principes.

Ainsi, dans le prélèvement du patient on préparera l'ARN (ou l'ADN) par des méthodes connues qui pourront dépendre de la nature du prélèvement (sang périphérique ou biopsie par exemple).

La synthèse d'ADN complémentaire par transcription réverse est connue, de même que le procédé permettant de ne transcrire que les mARN messagers présents dans cet ARN total, au moyen d'une amorce poly(T).

L'amplification peut être réalisée par une méthode de type PCR, c'est-a-dire par la méthode PCR ou par l'une de ses nombreuses variantes.

Le procédé est mis en oeuvre, de préférence, par PCR (polymerase chain reaction) et sans addition d'aucun traceur radioactif, coloré ou fluorescent. On amplifie l'ADN, pouvant provenir aussi bien d'une prise de sang que d'un autre prélèvement ou biopsie, toutefois le prélèvement initial doit contenir un nombre suffisamment élevé de cellules pour que les résultats ultérieurs soient statistiquement' significatifs. L'amplification démarre par une étape de transcription reverse (ou à partir de l'ADN extrait des cellules prélevées). Après quoi, on divise l'échantillon et on procède à autant de réactions d'amplification séparées qu'il y a de couples d'oligonucléotides (V,C).

On subdivise alors chacun des échantillons de façon à pratiquer, pour chacun d'entre eux, autant de réactions dites d'élongation ou de "run-off" qu'il y a de segments J. Pour effectuer ces réactions, on utilise des oligonucléotides spécifiques de ces segments J qui, cette fois, sont marqués, soit avec un traceur radioactif, soit, plus avantageusement, avec un fluorophore ou par d'autres méthodes. Dans la réaction de "run-off", la polymérase recopie jusqu'au bout les segments amplifiés à partir de l'oligonucléotide J. Si ceux-ci sont hétérogènes en longueur, on obtient donc un ensemble de molécules marquées à leur extremité J et dont on peut mesurer la taille avec précision.

Cette mesure de taille peut être faite sur des gels de séquence d'ADN conventionnels si l'on a effectué un marquage radioactif ou, avantageusement, avec un séquenceur automatique, notamment capable de détecter les fluorophores. L'appareil est donc utilisé uniquement pour mesurer les longueurs et l'intensité des produits d'élongation. Bien entendu, puisqu'il existe quatre fluorophores commerciaux de "couleurs" différentes, on pourra, comme il est fait dans les determinations de séquence d'ADN, mais pour des raisons totalement différentes, mélanger les produits de quatre réactions différentes utilisant des fluorophores différents. Par exemple, il pourra être particulièrement utile de mélanger les produits de réactions homologues faites sur un échantillon témoin et marques avec une couleur donnée, avec ceux de trois échantillons à étudier. De cette façon, on obtient une comparaison directe des échantillons au témoin, mais d'autres schémas expérimentaux sont possibles. Certains appareils commerciaux pratiquent une analyse des quatre couleurs dans 24 pistes simultanément (soit 96 échantillons). L'analyse prend quelques heures et les résultats sont informatisés.

Grâce à l'utilisation d'un séquenceur, on peut simplifier la lecture des autoradiogrammes dans la séquence conventionnelle qui est longue. Dans le cas précis décrit dans l'exemple, il faut lire chaque expérience, mémoriser et interpréter plus de 1 000 éléments. Dans le cas d'un séquenceur automatique, l'information est immédiatement mise en mémoire et l'utilisation des logiciels existants ainsi que d'un logiciel écrit dans le cadre de l'invention permet d'obtenir la matrice des résultats.

On notera que les problèmes pratiques sont encore plus considérables pour le répertoire $\alpha\beta$ de la souris où il est question d'analyser quelque 60 000 éléments. Le problème du répertoire $\beta$ est totalement soluble selon la stratégie adoptée pour les répertoires $\gamma$ et $\delta$. Le problème du répertoire $\alpha$ est ardu (5 000 échantillons à analyser pour chaque répertoire) mais des stratégies simplificatrices peuvent être développées. Il en va de même pour les répertoires d'anticorps et les répertoires humains.

Parmi les appareils aisément adaptables, il faut citer le séquenceur modèle 373A commercialisé par la société Applied Biosystems et les modèles apparentés.

Grâce au logiciel élaboré dans le cadre de l'invention, les données sont organisées, soit dans une représentation à trois dimensions (éléments génétiques dans le plan, longueur dans la hauteur), soit dans un tableau à deux dimensions. Cette représentation est préférable en ce qu'elle permet, dans une matrice de points, de figurer l'abondance du produit de réaction (par exemple, le point est plus ou moins foncé selon l'intensité de la bande détectée par le séquenceur) et ensuite de comparer aisément deux matrices (deux répertoires différents, deux états du même répertoire, etc...)

Une amélioration possible consiste à introduire un quatrième paramètre permettant d'analyser encore plus finement le répertoire. Il faut rappeler que cette notion de résolution est capitale, si l'on veut pouvoir analyser des évolutions dans le répertoire actuel. En effet, plus le nombre de paramètres de mesure est important, plus la résolution est grande, et plus les chances de pouvoir saisir des variations fines du répertoire sont importantes. Ce quatrième paramètre donne accès, comme le troisième, à la diversité jonctionnelle. Il s'agit d'appliquer aux produits de "run-off" une méthode d'électrophorèse réalisée en gradient de température ou de tout autre facteur dénaturant, de sorte que les produits de même taille soient séparés selon leur plus ou moins grande homologie avec une sonde fixe (Riesner et al., 1989). C'est donc un accès indirect à là séquence des régions variables qui est recherché. Des expériences sont en cours en utilisant comme sonde les gènes réarrangés dans des hybridomes.

Les exemples ci-après sont destinés à mettre en évidence d'autres avantages et caractéristiques de la présente invention.

Sur les figures ci-annexées,
- la figure 1 représente une partie du répertoire T$\gamma\delta$ dans le thymus (T), la rate (R) d'une souris Balb/c adulte et le placenta (P) d'une souris F1 (C3H x C57/B16),
- la figure 2 représente l'analyse, sur gel dénaturant, des produits d'élongation obtenus avec différentes amorces J$\beta$ sur de l'ADN extrait de ganglions amplifié par PCR avec des amorces spécifiques des segments V$\beta$ et C$\beta$ :

A et B :     souris B10.A immunisées avec le cytochrome C de pigeon:

        1 = non immunisées

        2 = après immunisation primaire

        3 = restimulation in vitro, après immunisation primaire ;

C :     souris C3H immunisées par le cytochrome C de pigeon:

        4, 5, 6 = trois souris différentes non immunisées

        7 = restimulation in vitro après immunisation primaire d'un groupe de souris,

les chiffres indiqués à droite ou à gauche des figures correspondent à la taille (en nucléotides) des produits allongés obtenus pour une combinaison V$\beta$ -J$\beta$ productive.

## Matériels et méthodes

### Oligonucléotides

Les oligonucléotides utilisés ont été synthétisés sur un automate 381A DNA Synthesier (Applied Biosystems). Le décrochage de la colonne et le déblocage des fonctions phosphates (protégées par un groupement béta-cyanoéthyl) s'effectuent en injectant dans la colonne 1,5 ml d'ammoniaque 28 % à raison de 0,5 ml par 30 mn. L'éluat est incubé pendant 12 heures à 56°C afin de déprotéger les bases de l'oligonucléotide (élimination des groupements benzoyl et isobutyril). Après lyophilisation, l'oligonucléotide est resuspendu dans de l'eau, à une concentration de 50 $\mu$M.

### Souris, cellules

Souris : Toutes les souches utilisées (Balb/c, Balb/b, DBA/2, CB 20, SJL et C3H/He) proviennent de l'animalerie de l'Institut Pasteur. Lorsque des accouplements ont été réalisés, le premier jour de gestation, jour où le bouchon vaginal est détecté, est appelé jour 0.

Cellules : Les hybridomes utilisés (S15, T14, T16, T18) résultent de la fusion de thymocytes d'une souris C3H/He adulte avec le partenaire BW5147$\alpha^-\beta^-$ (mutant du thymome BW5147 d'AKR, n'exprimant plus le récepteur $\alpha\beta$.

### Préparation d'ARN

L'ARN de tissus et de cellules en culture a été préparé selon trois méthodes différentes.

Première méthode, utilisée pour préparer l'ARN de cellules en culture : les cellules sont centrifugées ; le culot obtenu est resuspendu dans 10 ml d'une solution NaCH$_3$COO - NaCl 10 mM pH 5, 1 % SDS. Après incubation 2 mn à 4°C, 10 ml d'une solution de phénol non tamponnée, à 65°C sont ajoutés. L'ensemble est agité fortement pendant 5 mn ; après centrifugation 10 mn à 3 000 tour/min (rpm), la phase aqueuse est prélevée, remélangée à un volume de chloroforme-alcool isoamylique. Après centrifugation, l'ARN contenu dans la phase aqueuse est précipité en ajoutant 0,1 volume d'acétate de sodium 3 M pH 5,2 et deux volumes d'éthanol.

Deuxième méthode, dite méthode AGPC (Acid Guanidinium thiocyanate-Phenol-Chloroform) décrite dans Analytical Biochemistry, vol. 162, 1987, pp. 156-159: utilisable aussi bien pour préparer l'ARN d'un culot de cellules ou d'un organe, cette méthode permet d'éviter dans une très large mesure la dégradation des acides ribonucléiques susceptible d'intervenir dès que les cellules sont lysées. Cependant, des traces d'ADN subsistent dans la préparation, qui peuvent être gênantes dans certaines applications.

La troisième méthode élimine cette contamination par l'ADN en exploitant la différence de densité des acides ribo- et déoxyribonucléiques. La séparation de ces deux espèces est réalisée par ultracentrifugation du lysat en présence d'un gradient discontinu de CsCl: l'ADN est retenu à l'interface du gradient, tandis que l'ARN, plus dense, précipite. Cette méthode permet donc de récupérer à la fois l'ARN et l'ADN d'un tissu ou d'un culot cellulaire. Brièvement, une solution 4M de guanidine thiocyanate est préparée, contenant 0,5 % de Na N-laurylsarcosine, 25 mM EDTA, 0,13 % d'anti-foam A. Le gradient de CsCl est composé de 3 ml d'une solution 5,7 M de CsCl, 25 mM de Na acétate pH 5,2, 10 mM EDTA, et de 0,7 ml d'une solution 2,4 M de CsCl, 25 mM de Ma acétate pH 5,2, 10 mM EDTA. Le tissu est broyé dans 7 ml de la solution 4 M de guanidine thiocyanate à l'aide d'un homogénéiseur potter, le lysat obtenu est centrifugé 10 mn à 8 000 tour/min dans un rotor HB-4 pour éliminer les particules solides. Le surnageant est déposé délicatement sur le coussin de CsCl : l'ensemble est mis à centrifuger 24 heures dans un rotor SW 41 à 30 000 tour/min à 20°C. La phase liquide est alors éliminée (on peut lors de cette étape récupérer l'ADN situé à l'interface) et le culot est resuspendu dans 500 $\mu$l d'eau et précipité à - 20°C en ajoutant 1 ml d'éthanol et 50 $\mu$l de Na acétate 3 M, pH 5,2.

### Préparation d'ADN complémentaire

La synthèse de l'ADN complémentaire est réalisée à partir de l'ARN total préparé selon l'une des trois méthodes décrites plus haut. Le tampon utilisé est le même que celui de l'étape d'amplification par PCR (tampon Cetus). En effet, les différents tampons proposés ne se sont pas révélés d'une efficacité sensiblement supérieure (comparaison par mesure de l'incorporation de dCTP marqué au $^{32}$P) ; par contre, la concentration en Mg$_2^+$ de ces tampons ne permet pas de réaliser l'amplification par PCR sans autre étape intermédiaire. L'oligonucléotide utilisé comme amorce de la polymérisation du brin d'ADN complémentaire est un 15-mer poly-dT. Brièvement, 10 $\mu$g d'ARN total, lorsque celui-ci provient d'un organe de souris adulte, ou l'ARN de $10^5$ cellules provenant d'une culture ou d'un thymus fétal, sont mis à incuber 10 mn à 70°C dans 50 $\mu$l d'une solution 50 mM KCl, 10 mM Tris-HCl pH 8,2, 1,5 mM MgCl$_2$, 0,01 % gélatine, 0,1 mM EDTA, 5 $\mu$M pdT(15), 0,2 mM dNTP. Puis, après 5 mn sur la glace, 5 unités d'AMV RT (Proméga ou Boehringer) sont ajoutés, ainsi que 34 unités de RNasin (Pharmacia) ; le tout est incubé 1 h à 43°C, porté à 100°C pendant 5 mn.

## Amplification par la technique PCR

Les amplifications ont été réalisées sur les automates Perkin Elmer Cetus et Prem, selon la technique décrite (Saiki et al., 1988).

Pour amplifier l'ADN complémentaire le protocole utilisé fut le suivant : 5 $\mu$l de la solution préparée précédemment sont amplifiés dans 50 ou 100 $\mu$l d'une solution 50 mM KCl, 10 mM Tris-HCl, pH 8,2, 0,01 % gélatine, 0,2 $\mu$M en chacun des deux oligonucléotides utilisés, 2 u par 100 $\mu$l de Taq Polymérase (Beckman), 200 $\mu$M en chacun des nucléotides dATP, dGTP, dCTP et dTTP. La concentration en ion Mg$^{2+}$ utilisée varie de 2 à 2,5 mM. Le tout est recouvert de 50 $\mu$l d'huile minérale, chauffé 10 mn à 80°C, amplifié par 40 cycles (1 mn à 94°C, 1 mn à 60°C, 1 mn à 72°C) puis incubé 10 mn à 72°C.

Parmi les paramètres permettant de réduire le bruit de fond du procédé, il faut citer l'utilisation de concentrations optimales d'ions magnésium dans les étapes d'amplification ou d'élongation, ainsi on utilisera de préférence entre 1 et 3 mM d'ions magnésium.

## Marquage d'amorces, élongation par "run-off"

Les amorces J utilisées pour réaliser l'étape de "run-off" ont au préalable été radiomarquées par phosphorylation de l'extrémité 5' à l'aide d'un phosphate $^{32}$P. Le protocole est le suivant : 100 pmoles de l'oligonucléotide sont incubés dans 10 $\mu$l pendant 30 mn à 37°C dans le tampon d'amplification à une concentration de 2,5 mM en Mg$^{2+}$, en présence de 40 $\mu$Ci de $^{32}$P- ATP (soit 12 pmoles) et de 3 unités de T4 polynucléotide kinase. L'enzyme est ensuite inactivitée en portant le tout à 70°C pendant 10 mn.

L'élongation par "run-off" à partir des amorces J marquées est réalisée dans les conditions suivantes. 1 $\mu$l du mélange amplifié précédemment est dilué dans 10 $\mu$l d'une solution identique à celle qui a été utilisée pour l'amplification (tampon d'amplification avec la concentration optimale de Mg$^{2+}$ (2 à 2,5 mM), 200 $\mu$M en chacun des dNTP, 0,2 u de Taq Polymérase), à laquelle on ajoute l'amorce J marquée à la concentration de 1 $\mu$M (l'amorce J est donc un excès d'au moins 5 par rapport aux autres amorces V et C apportées par le $\mu$l du mélange amplifié). La réaction de "run-off" consiste en 3 mn à 94°C, 1 mn à 60°C suivi de 15 mn à 72°C.

## Electrophorèses

L'analyse des produits d'amplification et des séquences est réalisée par une électrophorèse sur gel de 400 $\mu$m d'épaisseur, de 6 % polyacrylamide (réticulation : 1 g de bisacrylamide pour 19 g d'acrylamide) dans le tampon TBE (Maniatis et al., 1982) en conditions de dénaturation (95 % formamide, 20 mM EDTA, bleu de bromophénol 0,25 %, xylène cyanol 0,25 %) et incubés 10 mn à 80°C. La migration est réalisée dans le tampon TBE sous un champ électrique de 4 000 Vm$^{-1}$, pendant 2 h ou 4 h pour l'analyse de séquence, et 5 à 6 h pour l'analyse de produits d'amplification.

## Exemple 1 - Etude de la diversité génétique de la chaîne $\gamma$ du récepteur T chez la souris

### Principe de la méthode

Pour chaque chaîne variable (V$\alpha$, V$\beta$, V$\gamma$ ou V$\delta$ pour les récepteurs T$\alpha\beta$ ou $\gamma\delta$), les oligonucléotides spécifiques sont synthétisés et permettent d'amplifier séparément les copies cADN des ARN messagers. Le tableau ci-après donne la liste des oligonucléotides utilisés dans l'analyse suivante. Les transcrits correspondants aux régions variables V$\gamma$1, V$\gamma$2... V$\gamma$7 sont amplifiés séparément, de même que ceux correspondant à V$\delta$1, V$\delta$2... V$\delta$9. Pour chaque produit d'amplification, une réaction spécifique des segments J est réalisée. Finalement, on mesure la taille des produits sur un gel de séquence. Ceci permet de décomposer les ARN messagers selon la longueur de la région variable (région N notamment) qui peut varier de 10 à 20 nucléotides environ. En outre, on peut repérer les ARN messagers qui sont hors-phase pour la traduction en protéine. En résumé, un échantillon est paramétré (1) pour l'emploi des chaînes V ; (2) pour l'emploi des segments J et (3) pour la taille.

La diversité génétique, pour les récepteurs $T\gamma\delta$ de la souris, provient du réassortiment de 15 éléments génétiques pour la chaîne $\gamma$ du récepteur et de 11 éléments de la chaîne $\delta$. Au cours de recombinaisons qui engendrent les gènes actifs de structure V-D-J-C, divers mécanismes opèrent aux jonctions de certains segments recombinés de sorte que la longueur du gène actif est variable dans des limites qui sont de 0 à 20 nucléotides environ pour les gènes $V\gamma$-$J\gamma$-$C\gamma$ et de 0 à 50 nucléotides environ pour les gènes $V\delta$-$J\delta$-$C\delta$.

Le répertoire du récepteur $T\gamma\delta$ a été paramètré par la détermination :
- des segments V,J et C qui sont utilisés,
- de la longueur du gène réarrangé.

On voit dans le tableau 1 que ceci représente plus d'un millier de mesures pour les récepteurs $T\gamma\delta$. Dans le cas des récepteurs $T\alpha\beta$, il s'agit de plus de 50 000 mesures.

- <u>Procédé expérimental</u>

Pour le répertoire des récepteurs $T\gamma\delta$ de la souris, sont synthétisés :
- 7 oligonucléotides spécifiques de chaînes $V\gamma$,
- 3 oligonucléotides spécifiques de chaînes $J\gamma$,
- 4 oligonucléotides spécifiques de chaînes $C\gamma$,
- 9 oligonucléotides spécifiques de chaînes $V\delta$,
- 2 oligonucléotides spécifiques de chaînes $J\delta$,
- 1 oligonucléotide spécifique de la chaîne $C\delta$.

Les méthodes de détermination des amorces sont connues, en particulier lorsqu'il existe de fortes homologies entre des séquences que l'on entend distinguer, par exemple $V\gamma1$, $V\gamma2$ et $V\gamma3$.

Le tableau 2 ci-annexé donne la liste des oligonucléotides utilisés.

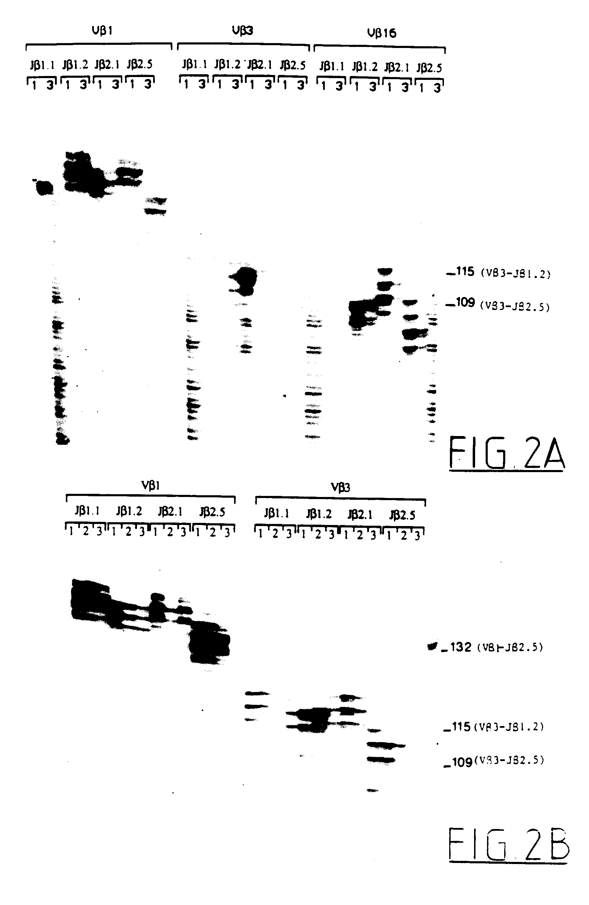

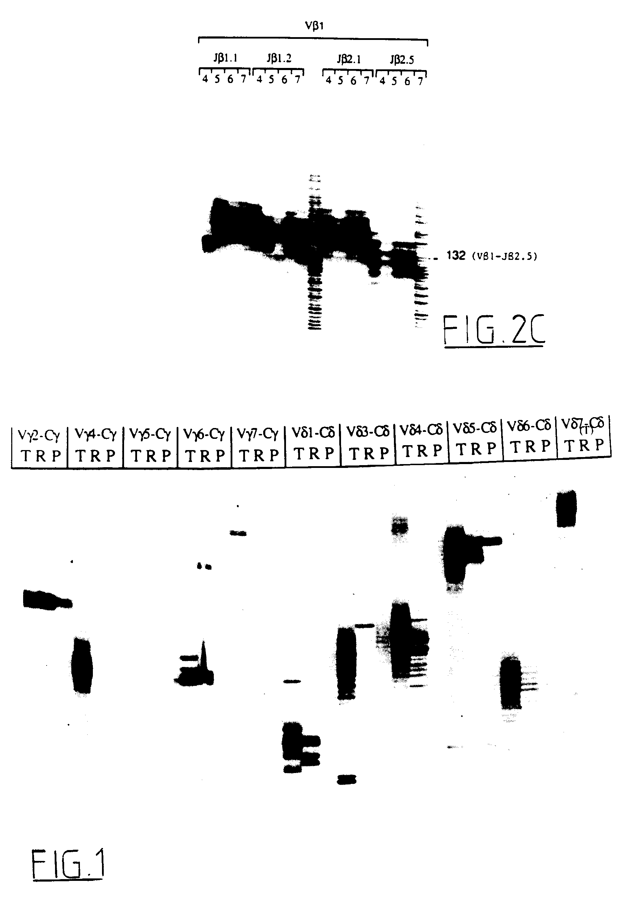

Les résultats obtenus sont répertoriés à la figure 1 sans l'usage J.

EP 0 566 685 B1

## Tableau 1

| | Immunoglobuline | | | Récepteur α β (1) | | Récepteur γ δ (2) | |
|---|---|---|---|---|---|---|---|
| | Chaîne V$_H$ | Chaîne V$\chi$ | Chaîne V$\gamma$ | Chaîne α | Chaîne β | Chaîne γ | Chaîne δ |
| **Répertoires murins** | | | | | | | |
| Segments V | 250-1000 | 250 | 2 | 100 | 25 | 7 | 10 |
| Segments D | 12 | 0 | 0 | 0 | 2 | 0 | 2 |
| Segments J | 4 + 1 | 4 + 1 | 4 | 50 | 12 | 3 | 2 |
| Régions C | 8 isotypes | 1 | 4 | 1 | 2 | 4 | 1 |
| Div. totale (3) | | $10^{11}$ | | $10^{15}$ | | $10^{18}$ | |
| **Répertoires humains (4)** | | | | | | | |
| Segments V | 100-200 | 80 | | 60 | 80 | 8 + 6 | 4 |
| Segments D | 4 | 0 | 0 | 0 | 2 | 0 | 2 |
| Segments J | 6 + 3 | 5 | 6 | 50 | 13 | 5 | 3 |
| Régions C | 10 isotypes | 1 | 6 | 1 | 2 | 2 | 1 |

(1) Wilson et al. (1988)
(2) Raulet (1989)
(3) Davis and Bjorkman (1988)
(4) Meindl et al. (1990).

Tableau 2

| | |
|---|---|
| Vγ1: | AGTTTGAGTATCTAATATATGTCT |
| Vγ2: | ACGACCCTTAGGAGGGAAGC |
| Vγ3: | TTGAGTATCTAATATATGTCGAG |
| Vγ2 et 3: | CGGCAAAAAACAAATCAACAG |
| Vγ4: | TGTCCTTGCAACCCCTACCC |
| Vγ5: | TGTGCACTGGTACCAACTGA |
| Vγ6: | GGAATTCAAAAGAAAACATTGTCT |
| Vγ7: | AAGCTAGAGGGGTCCTCTGC |
| | |
| Jγ1: | CTGCAAATACCTTGTGAAAA |
| Jγ2: | CTGCAAATACCTTGTGAAAG |
| Jγ4: | GAATTACTACGAGCTTTGTC |
| | |
| Cγ1: | TTTCAGCAACAGAAGGAAGG |
| Cγ2: | TCCAGGATAGTATTGCCATT |
| Cγ3: | GGAAATGTCTGCATCAAGCT |
| Cγ4: | GCTTGGGAGAAAAGTCTGAG |
| pan-Cγ: | CTTATGGAGATTTGTTTCAGC |
| | |
| Vδ1: | GGAATTCAGAAGGCAACAATGAAAG |
| Vδ2: | GTTCCCYGCAGATCCAAGCC |
| Vδ3: · | TTCCTGGCTATTGCCTCTGAC |
| Vδ4: | CCGCTTCTGTGTGAACTTCC |
| Vδ5: | CAGATCCTTGCAGTTCATCC |
| Vδ6: | TCAAGTCCATCAGCCTTGTC |
| Vδ7-R[2]: | GAAAGCTTCAGTGCAAGAGTC |
| Vδ7-T: | CGCAGAGCTGCAGTGTAAGT |
| Vδ8: | GCTACAGCACCCTGCACATC |
| | |
| Jδ1: | TCCACAGTCACTTGGGTTCC |
| Jδ2: | TCCACAAAGAGCTCTATGCC |
| | |
| Cδ: | CGAATTCCACAATCTTCTTG |

11

(1) Les nomenclatures utilisées sont celle de Heilig & Tonegawa (1986) pour le locus $\gamma$ et celle de Raulet (1989) pour le locus $\delta$. Le choix d'une partie des oligonucléotides provient directement de Takagaki et al. (1989 b).

(2) V$\delta$7-R est l'oligonucléotide spécifique du segment variable V$\delta$7 référencé dans Raulet (1989) ; V$\delta$7-T est l'oligonucléotide spécifique du segment variable V$\delta$7 référencé dans Takagaki et al. (1989 a).

### Exemple 2 - Analyse de la diversité génétique du récepteur T$\alpha\beta$

La diversité génétique de la chaîne $\beta$ du récepteur T sur des ganglions lymphatiques de souris B10.A, après immunisation par le cytochrome C de pigeon constitue un système de référence bien documenté. En particulier, d'après les travaux publiés, on sait que la chaîne $\beta$ de 75 à 80% des clones isolés après immunisation et restimulation in vitro par l'antigène, est composée du réarrangement V$\beta$3-J$\beta$1.2-C$\beta$.

Un échantillon d'ARN messager provenant de ganglions lymphatiques drainant de souris immunisées ou non, a été transformé en cADN. Celui-ci a été amplifié par PCR (Polymerase Chain Reaction) avec un couple d'oligonucléotides situés d'une part dans l'une des régions variables V$\beta$ et, d'autre part, dans la région constante. Cette opération a été répétée pour chaque région V$\beta$ (au nombre d'une vingtaine). A la suite de quoi, chaque réaction d'amplification, divisée en 12 échantillons (un pour chaque J$\beta$), a servi de matrice pour un cycle d'élongation avec l'un des 12 J$\beta$ radiomarqué.

La figure 2 jointe illustre une partie des résultats obtenus pour les couples V$\beta$1- V$\beta$3 et V$\beta$16-C$\beta$, réanalysés avec quatre J$\beta$ différents dont J$\beta$1.2., sur des souris B10.A (H-2a) dans deux expériences indépendantes (Figures A et B). Dans la figure A, pour le couple V$\beta$3-J$\beta$1.2, on voit apparaître une bande majoritaire dans les cellules T des ganglions restimulés in vitro par le cytochrome C de pigeons (figure A, canal 3). La taille des fragments constituant cette bande est en accord avec les résultats attendus (115 nucléotides pour V$\beta$3-J$\beta$1.2). Ces résultats sont reproductibles d'une expérience à l'autre (figure B) et, de plus, indiquent que la prépondérance du réarrangement V$\beta$3-J$\beta$1.2 est déjà détectable après la première immunisation (figure B, J$\beta$1.2, canal 2).

Il n'est pas exclu de pouvoir faire ce type d'analyse sur une même souris de manière à éviter le problème des variations minimes existant d'une souris à l'autre, ainsi que l'illustre la figure C (canaux 4 à 6).

### REFERENCES BIBLIOGRAPHIOUES

ABASTADO et al. (1984). Proc. Natl. Acad. Sci. USA 81, 5792-5796
ABASTADO et al. (1987). Proc. Natl. Acad. Sci. USA 84, 6496-6500
ASARNOW et al. (1989). Nature 341, 60-62
DAVIS and BJORKMAN (1988). Nature 334, 395-402
MANIATIS et al. (1982). Molecular Cloning: Laboratory Manual. Cold Spring Harbor Laboratory
MEINDL et al. (1990). Eur. J. Immunol. 20, 1855-1863
OKADA et WEISSMAN (1989). J. Exp. Med. 169, 1703-1719
RAJASEKAR et al. (1990). Proc. Natl. Acad. Sci. USA 87, 1767-1771
RAULET (1989). Ann. Rev. Immunol. 7, 175-207
RIESNER et al. (1989). Electrophoresis 10, 377-389
SAIKI et al. (1988). Science 239, 487-491
SINGER et al. (1990). EMBO J. (in press)
TAKAGAKI et al. (1989). Nature 339, 712-714
TONEGAWA (1983). Nature 302, 575-581
WILSON et al. (1988). Immunological Reviews 101, 149-172

**Revendications**

1. Procédé de description des répertoires d'anticorps (Ab) et de récepteurs des cellules T (TcR) du système immunitaire d'un individu, caractérisé en ce que :
   - à partir d'un prélèvement biologique, on effectue le cas échéant, la transcription réverse des ARNm qu'il contient,
   - on effectue ensuite, sur le produit de transcription (ou directement sur l'ADN extrait de l'échantillon), des amplifications séparées par une méthode de type PCR pour chaque couple d'amorce V,C, V correspondant à un segment variable du répertoire en cause et C s'hybridant au segment constant du répertoire étudié,
   - sur chacun de ces produits d'amplification on effectue, pour chaque segment J du répertoire marqué, une étape d'élongation utilisant comme amorce un oligonucléotide spécifique de ce segment J et le produit d'amplification comme matrice,
   - pour chaque produit d'élongation correspondant à un triplet (V,C)J ainsi obtenu on fait apparaître la taille des différents produits d'élongation,
   - la description des répertoires correspond pour chaque élément du répertoire à un triplet (V,C)J et à la taille de l'élément.

2. Procédé selon la revendication 1, caractérisé en ce qu'on fait apparaître les tailles des produits d'élongation par électrophorèse du mélange d'élongation et mise en évidence du marqueur.

3. Procédé selon l'une des revendications 1 et 2, caractérisé en ce que le marquage est radioactif, colorimétrique ou fluorescent.

4. Procédé selon l'une des revendications 1 à 3, caractérisé en ce que les étapes sont effectuées en présence de 1 à 3 mM d'ion Mg$^{2+}$.

5. Procédé selon l'une des revendications 1 à 4, caractérisé en ce qu'on mesure l'intensité de chaque signal de taille moléculaire afin d'obtenir une mesure quantitative de l'importance de la séquence correspondante.

6. Procédé selon la revendication 5, caractérisé en ce que l'intensité de chaque signal est évaluée par rapport à un étalon interne.

7. Procédé selon l'une des revendications 1 à 6, caractérisé en ce que les produits de même taille moléculaire sont séparés, en fonction de leur plus ou moins grande affinité pour une sonde fixe.

8. Procédé selon la revendication 7, caractérisé en ce qu'on effectue cette étape en électrophorèse à gradient de température ou de tout autre agent dénaturant.

9. Procédé selon l'une des revendications 1 à 8, caractérisé en ce que pour faire apparaître et décrire les tailles des produits d'élongation on utilise un appareil de séquençage automatique.

**Claims**

1. Method for describing the repertoires of antibodies (Ab) and of T-cell receptors (TcR) of an individual's immune system, characterized in that:
   - starting with a biological sample, reverse transcription of the mRNA which it contains is performed where appropriate,
   - on the transcription product (or directly on the DNA extracted from the sample), separate amplifications are then performed by a PCR type method for each primer pair V,C, V corresponding to a variable segment of the repertoire in question and C hybridizing with the constant segment of the repertoire under study,
   - on each of these amplification products, an elongation step is performed for each J segment of the labelled repertoire, using an oligonucleotide specific to this J segment as primer and the amplification product as template,
   - for each elongation product corresponding to a triplet (V,C)J thereby obtained, the size of the different elongation products is revealed,

- the description of the repertoires corresponds, for each element of the repertoire, to a triplet (V,C)J and to the size of the element.

2. Method according to Claim 1, characterized in that the sizes of the elongation products are revealed by electrophoresis of the elongation mixture and detection of the label.

3. Method according to one of Claims 1 and 2, characterized in that the labelling is radioactive, colorimetric or fluorescent.

4. Method according to one of Claims 1 to 3, characterized in that the steps are performed in the presence of 1 to 3 mM $Mg^{2+}$ ion.

5. Method according to one of Claims 1 to 4, characterized in that the intensity of each signal of molecular size is measured in order to obtain a quantitative measurement of the extent of the corresponding sequence.

6. Method according to Claim 5, characterized in that the intensity of each signal is evaluated relative to an internal standard.

7. Method according to one of Claims 1 to 6, characterized in that products of the same molecular size are separated in accordance with their greater or lesser affinity for a fixed probe.

8. Method according to Claim 7, characterized in that this step is performed using electrophoresis with a gradient of temperature or of any other denaturing agent.

9. Method according to one of Claims 1 to 8, characterized in that an automatic sequencing apparatus is used to reveal and describe the sizes of the elongation products.

**Patentansprüche**

1. Verfahren zur Aufstellung (Beschreibung) von Verzeichnissen (Listen) der Antikörper (Ab) und der Rezeptoren der T-Zellen (TcR) des Immunsystems eines Individuums, dadurch gekennzeichnet, daß man:
   - ausgehend von einer biologischen Probe, die mRNA, die sie gegebenenfalls enthält, einer reversen Transkription unterwirft,
   - anschließend mit dem Transkriptionsprodukt (oder direkt mit der aus der Probe extrahierten DNA) getrennte Amplifikationen durchführt nach einem Verfahrens vom PCR-Typ für jedes Primer-Paar V,C, wobei V einem variablen Segment des betreffenden Verzeichnisses (Liste) entspricht und C sich mit dem konstanten Segment des untersuchten Verzeichnisses (Liste) hybridisiert,
   - bei jedem dieser Amplifikationsprodukte für jedes Segment J des markierten Verzeichnisses (Liste) eine Verlängerungsstufe durchführt unter Verwendung eines für dieses Segment J spezifischen Oligonucleotids als Primer und des Amplifikationsprodukts als Matrix,
   - bei jedem auf diese Weise erhaltenen Verlängerungsprodukt, das einem Triplett (V,C)J entspricht, die Größe der verschiedenen Verlängerungsprodukte bestimmt, und
   - Verzeichnisse (Listen) aufstellt, die für jedes Element des Verzeichnisses (der Liste) einem Triplett (V,C)J und der Größe des Elements entsprechen.

2. Verfahren nach Anspruch 1, dadurch gekennzeichnet, daß man die Größe der Verlängerungsprodukte durch Elektrophorese des Verlängerungsgemisches und Nachweis des Markers bestimmt.

3. Verfahren nach einem der Ansprüche 1 und 2, dadurch gekennzeichnet, daß die Markierung radioaktiv, cholorimetrisch nachweisbar oder fluoreszierend ist.

4. Verfahren nach einem der Ansprüche 1 bis 3, dadurch gekennzeichnet, daß die Stufen in Gegenwart von 1 bis 3 mM $Mg^{2+}$-Ionen durchgeführt wird.

5. Verfahren nach einem der Ansprüche 1 bis 4, dadurch gekennzeichnet, daß man die Stärke jedes Signals der Molekülgröße mißt zur Gewinnung eines quantitativen Maßstabs für die Größe der

14

entsprechenden Sequenz.

6. Verfahren nach Anspruch 5, dadurch gekennzeichnet, daß die Stärke jedes Signals bewertet wird unter Bezugnahme auf einen internen Standard.

7. Verfahren nach einem der Ansprüche 1 bis 6, dadurch gekennzeichnet, daß die Produkte mit der gleichen Molekülgröße als Funktion ihrer mehr oder minder großen Affinität für eine feste Sonde voneinander getrennt werden.

8. Verfahren nach Anspruch 7, dadurch gekennzeichnet, daß man die Elektrophorese-Stufe mit einem Temperaturgradienten oder mit irgendeinem anderen denaturierenden Agens durchführt.

9. Verfahren nach einem der Ansprüche 1 bis 8, dadurch gekennzeichnet, daß man zum Nachweis und zur Beschreibung der Größen der Verlängerungsprodukte eine automatische Sequenzierungsvorrichtung verwendet.

FIG. 2A

FIG. 2B

16

FIG.2C

FIG.1